(19) Europäisches Patentamt / European Patent Office / Office européen des brevets

(11) **EP 2 339 350 A1**

(12) **EUROPEAN PATENT APPLICATION**

(43) Date of publication:
**29.06.2011 Bulletin 2011/26**

(51) Int Cl.:
***G01N 33/68*** *(2006.01)*

(21) Application number: **11156690.7**

(22) Date of filing: **25.03.2003**

(84) Designated Contracting States:
**AT BE BG CH CY CZ DE DK EE ES FI FR GB GR HU IE IT LI LU MC NL PT RO SE SI SK TR**

(30) Priority: **25.03.2002 US 366550 P**

(62) Document number(s) of the earlier application(s) in accordance with Art. 76 EPC:
**03712410.4 / 1 488 241**

(71) Applicant: **SENSE PROTEOMIC LIMITED**
**Yarnton**
**Oxford**
**Oxfordshire OX5 1PF (GB)**

(72) Inventors:
 • **Kozlowski, Roland**
  **Woodstock, Berkshire OX20 1DL (GB)**
 • **Blackburn, Jonathan**
  **Cambridge, CB3 0HS (GB)**
 • **Hayward, Rhian**
  **Maidenhead, Berkshire SL6 7RJ (GB)**
 • **Cameron, Grant**
  **Maidenhead, Berkshire SL6 7RJ (GB)**

(74) Representative: **Marshall, Cameron John**
  **Carpmaels & Ransford**
  **One Southampton Row**
  **London WC1B 5HA (GB)**

Remarks:
This application was filed on 02-03-2011 as a divisional application to the application mentioned under INID code 62.

(54) **Arrays**

(57)   Protein arrays that allow the analysis of differentially expressed proteins in parallel are disclosed. Methods for making such arrays, and their use for screening and/or evaluating the effect of chemical entities, including drug and therapeutic moieties, are described.

The arrays can also be used to screen for compounds, peptides, or proteins, which modulate the interaction between a protein and the differentially expressed protein.

**EP 2 339 350 A1**

**Description**

The present invention relates to protein arrays which allow for analysis of differentially expressed proteins in parallel.

A number of high throughput methods are currently used to catalogue the differential expression patterns of proteins within a given cell or tissue type, or between healthy, diseased and drug treated cells or tissues. For example, high throughput functional genomics tools such as DNA microarrays [1] are routinely used to generate large quantities of data on, for example, the transcriptomes associated with specific disease states [1] and rapid diagnosis methods based on DNA array technology are in now development [2]. At the protein level, 2-D gel electrophoresis [3] is now widely used to identify and catalogue proteins that are present or absent in different pathological states or cellular fractions. Examples of groups of differentially expressed proteins that are of interest include:

proteins expressed in different cellular compartments, eg the nucleus or the cytoplasm;
proteins that are eventually translocated out of the cell entirely, ie the secreted proteins;
proteins whose expression levels alter in response to an external stimulus such as drug treatment;
proteins whose expression levels alter between healthy and diseased states.

However, while the list of protein constituents of a tissue or cellular compartment at any one moment allows the gathering together of protein players in disease or specific cellular phenotype/function, it does not provide in isolation any functional data on interaction, activities and pathways conferring disease or function.

Currently there is a paucity of such functional data available to describe the exact phenotypic consequences of differential protein expression and it is the study of the protein compliment of a cell of tissue, known as proteomics, which will ultimately reveal this information. What is required then is knowledge of not only the relative protein expression levels but also the functional effects or consequences of the differentially expressed proteins, but currently no method exists to provide the latter in a high throughput manner since proteins are typically analysed for function in a one-by-one, serial manner.

Currently, differentially expressed proteins can be identified by two dimensional gel electrophoresis. Although this technique provides information on proteins present and absent and relative protein expression levels it does not provide functional information on the role of each differentially expressed molecule. Furthermore, this technique does not enable detection of proteins that are expressed in low copy number [4] and many proteins fail to be detected due to limitations in visualising differentially expressed molecules in gels by current staining techniques. The identification of small quantities of proteins, even by mass spectrometry, is not possible if the proteins cannot be detected and therefore are not identified as differential expressed.

Once differentially expressed proteins have been identified, protein-protein interactions can be studied on a one-by-one basis using yeast two-hybrid methods [Y2H; ref 5]. Y2H has several disadvantages for building a comprehensive picture of differentially expressed protein pathways. At present, Y2H can be used to identify the interacting partners for one "bait" protein at a time. This limits the input to a relatively small number of proteins and therefore does not allow proteome-scale experiments. Y2H also does not permit multiplexing as it does not allow screening of several protein baits in the same experiment in order to identify protein complexes that may form between them, which may influence further individual interactions. In addition, any non-yeast proteins that are screened for binding to 'baits' may not be assayed in their native state, as expression and subsequent interactions occur in yeast. Finally, Y2H is not amenable to the study of entities that block or antagonise protein-protein interactions. This type of study is an essential criterion for assessing the importance of differentially expressed protein pathways for drug discovery. Small molecules that disrupt protein-protein interactions must be assayed by an independent method in this case.

Pull down assays/immunoprecipitations can be used to study protein-protein interactions. The test proteins are affinity tagged *in vitro* or *in vivo* and are then allowed to interact with other proteins within a solution or within the cell before being precipitated, via their tag, for identification of binding [6]. Although this method allows identification of many interacting protein partners at one time (complexes) providing additional information to Y2H, it also requires cell-based assaying that limits the throughput of the system. Finally, pull down assays or immunoprecipitations do not allow for the controlled testing of the effects of peptides or antibodies on the interactions observed as the experiments are conduced within the context of a whole cell and degradation and/or failure of local trafficking of the test molecule could prevent contact with the interacting proteins.

A number of recent publications [e.g. ref 7] have shown that arrays of functional proteins allow the individual members of an array to be screened simultaneously under identical conditions. This allows highly parallel and rapid experiments compared with other techniques, leading to directly comparable results across many proteins. These qualities set protein arrays apart from other assays for protein function, such as Y2H and immunoprecipitations/pulldowns, where the cellular compartmentalisation that is implicit in these other methods effectively divides each protein collection into individual proteins and thus individual assays.

Results obtained from the interrogation of arrays of the invention can be quantitative (e.g. measuring binding or catalytic constants $K_D$ & $K_M$), semi-quantitative (e.g. normalising amount bound against protein quantity) or qualitative (e.g. functional vs. non-functional). By quantifying the signals for replicate arrays where the ligand (e.g. DNA, protein, antibody, peptide or small molecule) is added at several (for example, two or more) concentrations, both the binding affinities and the active concentrations of protein in the spot can be determined. Exactly the same methodology could be used to measure binding of drugs to arrayed proteins.

For example, quantitative results, $K_D$ and $B_{max}$, which describe the affinity of the interaction between ligand and protein and the number of binding sites for that ligand respectively, can be derived from protein array data. Briefly, either quantified or relative amounts of ligand bound to each individual protein spot can be measured at different concentrations of ligand in the assay solution. Assuming a linear relationship between the amount of protein and bound ligand, the (relative) amount of ligand bound to each spot over a range of ligand concentrations used in the assay can be fitted to equation 1, rearrangements or derivations.

$$\text{Bound ligand} = B_{max} / ((K_D/[L])+1) \qquad \text{(Equation 1)}$$

[L] = concentration of ligand used in the assay

An estimation of inhibition (IC50) by a compound on an interaction can also be measured with replicate protein array assays.

Differentially expressed proteins represent a specific subset of the overall collection of proteins in a given cell, tissue or organism that may have particular clinical and pharmaceutical relevance. There is unlikely to be any significant sequence, structural or functional similarity across such a set of proteins and, as a result, protein arrays consisting of this protein group would represent a highly versatile tool with potential applications in drug target identification and validation processes as well as in drug selectivity screens and in delineation of differentially expressed protein-protein interaction maps. However, for such applications to be viable, the differentially-expressed proteins on the array need to be correctly folded such that they are likely to retain many if not all aspects of their natural function; such an array has not previously been described and is not obvious from the prior art for a number of fundamental reasons:

   i) arraying of proteins in a functional form has rarely been scaled up for large numbers since it is entirely dependent upon the ability to first generate an appropriate collection of expressed, purified and functional proteins;
   ii) conventionally this would require prior knowledge of the identity of the differentially expressed proteins and this is itself rarely known with any degree of completeness;
   iii) methods such as subtractive hybridisation have not been combined with expression studies on differential regulated transcripts;
   iv) proteins isolated using 2-D gel electrophoresis methods are typically denatured during the separation process. Refolding of such proteins to restore function is unlikely to be successful in a high throughput format since typically each individual protein will require highly specific refolding conditions, if refolding is possible at all;
   v) high throughput column-based purification of differentially expressed proteins has not been described;
   vi) in any event, proteins obtained by (iii), (iv) or (v) would lack a common handle, such as an affinity tag, through which they could be immobilised onto a single surface without impairing function.

In vitro screening of protein interactions in an array format has been demonstrated. In it's simplest form, microarrays have been generated from immunoglobulin molecules in order to capture proteins from solution [8-10]. These antibody arrays provide miniaturisation of the ELISA assay and enable high throughput analysis of e.g. cell lysates, serum samples or recombinant protein mixtures. A second example of protein array types is the antigen array, used to identify auto-antibodies in serum samples [11]. In these cases, the antigens are arrayed on a denaturing surface, making all linear epitopes available for antibody binding but destroying the native form of the arrayed molecules. Two examples of protein arrays in which the proteins were arrayed to retain correct folding and function have recently been described. In the first example, a 'proteome on a chip' was created for the relatively small yeast genome [12], enabling the researchers to identify activities based on binding to the proteins in their native conformations. In the second example, a small array of protein kinases has also been created [8]. In addition, arrays of randomly selected, functional proteins that have been specifically tagged at the N- or C-terminus have been created and interrogated to identify interacting partners such as DNA and small molecules have been described [13]. There has been no description to date of an array of folded, differentially expressed proteins.

Thus, there is still a lack of high throughput tools for the functional study of differentially expressed proteins and also a lack of tools to assay the effects of drug molecules on these functions in parallel. As the numbers of differentially expressed proteins may approach the hundreds, if not the thousands, a highly parallel method of functional analysis is needed that does not require antibodies, gels or beads to perform. The present invention is based on protein arrays and will have applications for specific drug target identification and validation and drug selectivity. Additional applications will include screening of interacting species, eg other proteins, to identify differentially expressed protein pathways.

The present invention is a collection of proteins, which together represent a proteomic footprint of a specific disease state or cellular compartment. The individual proteins in said collection are affinity tagged, purified and in a folded conformation. In addition, the individual proteins are spatially separated and specifically immobilised on to a surface in an array format such that the folded state of the individual proteins is unlikely to be perturbed.

Thus, in a first aspect, the present invention provides a protein array comprising a surface upon which are deposited at spatially defined locations at least two protein moieties characterised in that said protein moieties represent at least part of a set of proteins which are differentially expressed.

As already indicated herein, examples of such groups of differentially expressed proteins include:

proteins expressed in different cellular compartments, eg the nucleus or the cytoplasm;
proteins that are eventually translocated out of the cell entirely, ie the secreted proteins;
proteins whose expression levels alter in response to an external stimulus such as drug treatment;
proteins whose expression levels alter between healthy and diseased states.

A protein array as defined herein is a spatially defined arrangement of protein moieties in a pattern on a surface. Preferably the protein moieties are attached to the surface either directly or indirectly. The attachment can be non-specific (e.g. by physical absorption onto the surface or by formation of a non-specific covalent interaction). In a preferred embodiment the protein moieties are attached to the surface through a common marker moiety appended to each protein moiety. In another preferred embodiment, the protein moieties can be incorporated into a vesicle or liposome which is tethered to the surface.

The number of proteins attached to the arrays of the invention will be determined, at least to a certain extent, by the number of proteins that occur naturally or that are of sufficient experimental, commercial or clinical interest. An array carrying one or two proteins would be of use to the investigator. However in practice and in order to take advantage of the suitability of such arrays for high throughput assays, it is envisaged that 1 to 10000, 1 to 1000, 1 to 500, 1 to 400, 1 to 300, 1 to 200, 1 to 100, 1 to 75, 1 to 50, 1 to 25, 1 to 10 or 1 to 5 such proteins are present on an array.

A surface as defined herein is a flat or contoured area that may or may not be coated/derivatised by chemical treatment. For example, the area can be:

a glass slide,
one or more beads, for example a magnetised, derivatised and/or labelled bead as known in the art,
a polypropylene or polystyrene slide,
a polypropylene or polystyrene multi-well plate,
a gold, silica or metal object,
a membrane made of nitrocellulose, PVDF, nylon or phosphocellulose

Where a bead is used, individual proteins, pairs of proteins or pools of variant proteins (e.g., for "shotgun screening" - to initially identify groups of proteins in which a protein of interest may exist; such groups are then separated and further investigated (analogous to pooling methods known in the art of combinatorial chemistry)) may be attached to an individual bead to provide the spatial definition or separation of the array. The beads may then be assayed separately, but in parallel, in a compartmentalised way, for example in the wells of a microtitre plate or in separate test tubes.

Thus a protein array comprising a surface according to the invention may subsist as series of separate solid phase surfaces, such as beads carrying different proteins, the array being formed by the spatially defined pattern or arrangement of the separate surfaces in the experiment.

Preferably the surface coating is capable of resisting non-specific protein absorption. The surface coating can be porous or non-porous in nature. In addition, in a preferred embodiment the surface coating provides a specific interaction with the marker moiety on each protein moiety either directly or indirectly (e.g. through a protein or peptide or nucleic acid bound to the surface). A variety of surfaces can be used, as well as surfaces in microarray or microwell formats as known in the art.

In general, the individual members of the protein array each will contain an identical affinity tag through which they can be immobilised, thereby minimising the risk of perturbing the function of the arrayed proteins through non-specific contact with the surface.

The array format then allows the collection of differentially expressed proteins to be interrogated with a wide range of functional assays in a highly parallel manner in order to identify, for example, interactions with other proteins. In particular, the array provides a tool for screening individual entities that may block or antagonise these interactions across many hundreds or thousands of proteins simultaneously.

The collection of soluble, purified, and immobilised proteins making up a differential display array might be generated in several ways. After transcriptomic analysis such as subtractive hybridisation or cDNA microarray analysis or proteomic analysis such as two dimensional gel electrophoresis, differentially expressed cDNAs or proteins could potentially be identified by their sequence. Conventional cloning could then be used to express these genes as proteins for arraying. Alternatively the methods described in ref. 13 can be used for sequence-independent cloning and expression of a subtracted cDNA library. This latter approach does not rely on prior knowledge of the identity of the differentially expressed genes or proteins. It can therefore clearly be used to generate a set of tagged, purified, folded, differentially expressed proteins (and hence an array comprising any such set) from, for example, any given diseased or drug-treated cell or tissue since the differentially expressed cDNAs can be readily isolated by numerous methods, such as subtractive hybridisation, that are known in the art. In addition, cDNAs encoding collections of secreted proteins can be isolated using signal-trap systems known in the art.

Subtractive hybridisation represents one preferred method for isolating the set of differentially expressed cDNAs, and in particular readily enables identification of both up-regulated and down-regulated proteins.

The methods described herein are not restricted to use of recombinant protein expression hosts. As known in the art, a range of different expression hosts including bacteria, yeast, *Drosophila spp.* or mammalian cells could be transformed with vectors (plasmid or virus) containing the differentially expressed cDNAs fused in frame to an affinity tag. Similarly, the construction of the protein array is not constrained by the type or number of affinity tags used, although the proteins should be deposited in such a way on the solid surface as to retain their correct folding.

To make an array of purified, discrete protein spots, cell lysates can be deposited directly on to the surface, performing purification and immobilisation via the affinity tag in one step, and creating an array. Alternatively proteins can be purified via a first affinity tag and then arrayed for immobilisation through a second tag.

The protein arrays of the present invention can be probed with potential interacting partners such as crude cell lysates, other (individual) proteins, nucleic acids or small molecules/drugs to identify previously unknown interactions. The differential display protein array method allows the detection and characterisation of interactions by fluorescence, colorimetric and chemiluminescence techniques (microarray format). In addition, the array contains individually, highly purified proteins enabling each protein interacting with a differentially expressed protein on the array to be identified by mass spectrometry (microwell plate format) and surface plasmon resonance methods (fluidics chamber format). The method is not format dependant and does not require antibodies, beads, gels or recombinant host organisms in the assays, separating it from current methods.

Thus, in a second aspect, the present invention provides a method of making a protein array comprising the steps of:

a) providing DNA coding sequences which code for two or more proteins from a set of differentially expressed proteins;
b) expressing said coding sequences to provide individual proteins;
c) purifying said proteins; and
d) depositing said proteins at spatially defined locations on a surface to give an array.

Thus, utilising the information from a two dimensional gel electrophoresis, subtractive hybridisation or DNA microarray experiments for functional analysis of proteins to identify genes that are transcribed in a diseased tissue or cell that are not transcribed in the normal tissue of cell or genes which are expressed in a discrete cellular compartment or which are secreted cell proteins, and using a protein array format to display the encoded proteins, identified through these methods, such that they retain function (folding and solubility) enables the performing of highly parallel experiments on a 'proteomic footprint of differentially expressed genes'.

This also allows for 'on-array' pull down and identification of multiple protein binding partners to each array member. Binding of one or many proteins from single or complex mixtures enables multiple proteins contained, for example, in multiprotein complexes, to bind and be identified via a single arrayed, differentially expressed protein. Simultaneous creening of the affects of new chemical entities, that may disrupt or antagonise such interactions, on hundreds or thousands of proteins under the same experimental conditions are possible

Thus, in a third aspect, the present invention provides a method for screening/evaluating the effect of a drug/chemical entity/therapeutic moiety which comprises the step of bringing said drug/chemical entity/therapeutic moiety into contact with an array of the invention.

The invention will now be described with reference to the following figures:

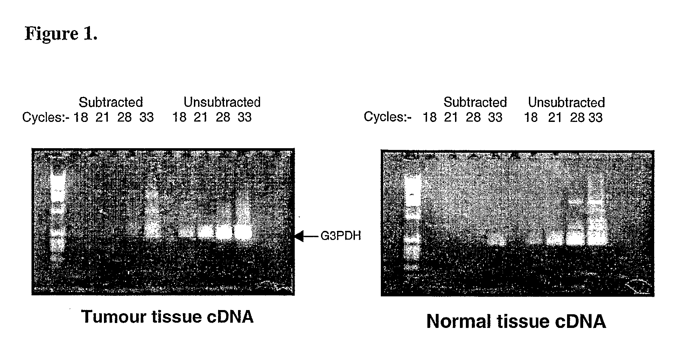

**Figure 1** Analysis of efficiency of cDNA subtraction, by detection of housekeeping gene glucose 3 phosphate dehydrogenase (G3PDH). CDNA subtraction was performed on both breast tumour cDNAs and normal tissue cDNAs by presentation of the other as the DRIVER population. Remaining subtracted cDNAs were analysed for the removal of G3PDH (expected if efficiency is high) by PCR amplification of subtracted (and un-subtracted cDNA's for comparison) using G3PDH-specific oligonucleotide primers and varying numbers of amplification cycles to product a semi-quantitative result. The specific G3PDH amplicons are indicated.

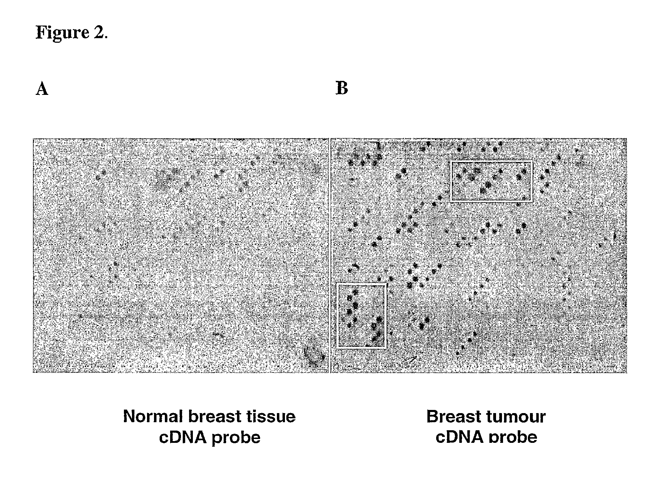

**Figure 2** Analysis of the representation of subtracted breast tumour cDNA clones in normal breast tissue (A) and breast tumour tissue (B) by southern blotting. *E. coli* colonies containing 384 subtracted breast tumour cDNA clones were robotically deposited on nylon membranes to grow overnight on agar plates at 37°C. The membranes were processed following a standard protocol (see Materials and Methods) and the southern blots then probed with DIG-labelled cDNAs generated from normal or tumour tissues. After stringent washing, blots were developed using ECL reagents (Amersham Biosciences, UK) and exposed to x-ray film. White boxes indicate cDNA clones that are exclusively or overly expressed in breast tumour tissue relative to normal breast tissue

## Example 1

Differentially expressed cDNAs containing a signal peptide sequence are expressed and arrayed in a format compatible with parallel analysis. The method described here involves transformation of a yeast or mammalian expression host with a library of plasmids containing small fragments of genomic DNA that are fused in frame to a reporter gene construct in a plasmid. When the reporter protein is expressed and translocated to the outside of the cell it can be detected and the gene associated with the identified signal peptide can be identified, cloned and expressed for protein arraying.

### Signal Peptide Trap

A sample of cDNA (from a library or direct synthesis source) is fragmented by sonication, blunted ended using appropriate enzymatic modification [14] and cloned en masse into an expression vector. This plasmid contains a reporter protein, such as for example, invertase or plasminogen. The reporter gene is expressed as protein if fused in frame with a piece of cloned cDNA. If a signal peptide sequence is contained within the cloned fragment then the protein can be detected when secreted outside of the transformed expression host cell using an appropriate assay [15].

### Capturing full length cDNAs for secreted proteins.

The cDNA fragments containing the signal peptide are used to capture the corresponding full-length cDNAs from a cDNA library using ClonCapture cDNA Selection kit (Clontech) following manufacturer's instructions. The captured plasmid library is then amplified through transformation of XL-1 blue *E. coli* and plasmids then extracted (Qiagen Maxi Plasmid Purification kit).

### Expression of soluble, recombinant secreted proteins

Full length cDNA clones are captured as above. The procedure described in ref 13 is then carried out on the pooled plasmid library encoding the secreted protein set and the final ligation mix is recovered by transformation of XL-1 blue *E. coli*. The secreted proteins are now encoded in the new library as fusions to a C-terminal green fluorescent protein-biotin carboxyl carrier protein [GFP-BCCP; refs. 16 & 17] tag DNA sequence. Restriction digestions are then performed [14] to remove the newly cloned inserts, together with the DNA encoding the tag, from the library vector and these are then ligated (directionally) into a suitable expression vector for use in yeast cell lines. Once transformed, these recombinant host cells are induced under appropriate conditions and those clones which express protein tagged proteins are identified. The secreted, tagged proteins are then purified and immobilised into an array in a single step from the cell culture (see below).

## Example 2

Proteins from a subtracted set of human breast tumour tissue cDNAs are arrayed in a format suitable for parallel functional analysis. The method described here involves transformation of a bacterial expression host with a library of plasmids containing differentially expressed cDNAs that are fused in frame to an affinity tag using the methodology described in ref 13. The proteins remain folded and active when expressed. Cell lysates are deposited on to a solid surface, performing purification and immobilisation via the affinity tag in one step, and creating an array of differentially expressed proteins.

**Subtractive hybridisation of breast tumour and normal breast tissue cDNAs**

Total RNA (1$\mu$g) from female breast tumour tissue (invasive ductal carcinoma) and matched normal breast tissue (AMS Biotech) is used to synthesise first strand cDNA using the SMART cDNA Synthesis Kit (Clontech) following manufacturer's instructions. A subtractive hybridisation is then performed using PCR-Select Subtractive Hybridization kit (Clontech) with breast tumour cDNAs as 'Tester' and normal tissue cDNAs as 'Driver', and *vice versa* (as a control experiment) following manufacturer's instructions, to generate a subtracted set of cDNAs.

**Testing efficiency of subtraction**

The subtracted set of tumour-associated cDNA fragments is cloned *en masse* into the TOPO TA cloning vector (Invitrogen). XL1 blue *E. coli* (Stratagene) are transformed with ligation mix and 100 cDNA clones are picked, plasmids extracted (Qiagen Mini Plasmid Purification kit) and cDNA inserts are sequenced using M13 reverse universal primer (LARK Technologies, U.K.). PCR amplification of a housekeeping gene, glucose 3 phosphate dehydrogenase, can be attempted from the subtracted cDNA set using G3PDH-specific primers under standard conditions: 95°C 1 min, then varying numbers of cycles of 95°C 30 s, 58°C 30 s, 68°C 1.5 min. Amplicons are analysed by 1.2% agarose gel electrophoresis (Figure 1).

Southern blotting (used standard techniques of bacterial colony blotting [14]) is used to assess the abundance of subtracted set cDNAs (designated the probe) in normal breast tissue and breast tumour tissue cDNA pools (the target; Figure 2).

**Capturing full length breast tumour-associated cDNA clones**

The subtracted set of cDNA fragments is used to capture the corresponding full length cDNAs from a human heart cDNA library (Clontech) and a human breast tumour cDNA plasmid library (unpublished) using ClonCapture cDNA Selection kit (Clontech) following manufacturer's instructions. The captured plasmid library is then amplified through transformation of XL-1 blue *E. coli* and plasmids then extracted (Qiagen Maxi Plasmid Purification kit).

**Cloning and expression of tagged and folded breast tumour-associated proteins**

*Experiment 1*

The subtracted breast tumour cDNA plasmid library is used to transform XL-1 blue *E. coli.* 384 bacterial colonies can be picked at random to analyse the cDNA inserts by restriction fragment length polymorphism patterns (14; data not shown). Based on RFLP pattern in this example, 96 different cDNA clones were selected and subjected (individually) to the procedure described in ref 13. Each resultant modified cDNA is cloned by ligation with a suitably prepared equimolar pool of three plasmid vectors designated pIFM101-A, -B, & -C; these vectors each contain a C-terminal green fluorescent protein-biotin carboxyl carrier protein [GFP-BCCP; refs. 16 & 17] tag DNA sequence in a unique reading frame relative to their common 3'-cloning sites (i.e. all three possible reading frames of the GFP-BCCP tag relative to a common 3'-cloning sites are represented in a pool of these three vectors). DNA from each ligation is then used to transform *E. coli* XL-1 blue. After overnight growth on solid media, protein expression can be induced at 30°C for ca. 4 hrs by addition of IPTG. Green fluorescent colonies (indicating expression of a soluble, GFP-BCCP-tagged fusion) are picked and the plasmids subjected to PCR amplification of the cDNA inserts (using plasmid-specific primers). DNA sequencing, using universal M13 reverse primer, is used to identify the cloned cDNAs.

*Experiment 2*

The complete subtracted breast tumour cDNA plasmid library is used to transform XL-1 blue *E. coli.* In this example $3 \times 10^5$ bacterial colonies are scraped from agar plates, mixed and the plasmids extracted (Qiagen Maxi Plasmid Purification kit). The procedure described in ref 13 is carried out on the pooled plasmid library and the final ligation mix used to transform XL-1 blue *E. coli.* 244 green fluorescent colonies were picked (out of a total of 9,760 green fluorescent colonies). Southern blotting is carried out on the clones from the 244 bacterial colonies [14] with a probe consisting of 8 common genes. 184 different cDNA clones were consolidated and PCR was used to amplify the cDNA inserts which were then sized on agarose gels. 57 cDNA clones which contained cDNAs >300 bp, were identified. Extrapolation to the total number of colonies showed that the total library contains 2,262 colonies expressing soluble proteins of >12.5kDa fused in frame to the GFP-BCCP tag.

Western blot analysis [14] of proteins expressed from final cDNA clones in both experiments confirmed, in this example, expression of 82 soluble, biotinylated proteins of >12.5kDa. Final DNA sequencing of these cDNAs (Table 1) is carried out using universal M13 reverse primer.

**Analysis of differentially expressed proteins**

Quality control experiments confirm that the initial subtraction process and subsequent modification yields a diverse collection of cDNAs, some of which are associated with breast cancer. The efficiency of subtraction is high as analysis of the subtracted library firstly reveals that very little cDNA encoding the housekeeping gene G3PDH remains after the subtractive hybridisation when it is performed in either direction (Figure 1). This compares to a starting amount of this cDNA that was detected in both unsubtracted samples after only 18 cycles of PCR amplification. Secondly, subtraction of normal cDNAs from breast tumour cDNAs does not introduce any significant bias, as DNA sequencing (data not shown) reveals that only 6% of the breast tumour subtracted cDNA set are redundant (i.e. appeared more than once). Southern blotting confirms that most cDNAs in the breast tumour subtracted cDNA set are, as expected, over-abundant in the starting breast tumour cDNA sample whilst some cDNAs are specific to a tumour cDNAs sample (Figure 2).

DNA sequence analysis of 56 unique clones that expressed soluble, tagged proteins (drawn from Experiments 1 & 2) reveals several proteins already known to be associated with cancer such as BCL-like 2 gene [18] and hypoxia-inducible gene 1 [19], as well as several proteins for which there is no functional or disease-associated information currently available such as I.M.A.G.E. cDNA clones and unknown/unannotated genes. Appearance of the latter proteins in the differentially displayed set contribute immediately to information on several previously uncharacterised genes and allow the functional assay of the encoding proteins in the context of this pathology.

**Examples 1 and 2**

**Arraying of soluble, recombinant proteins**

Examples 1 & 2 described above both create collections of solubly expressed, folded, C-terminally tagged, differentially expressed proteins in a form directly suitable for immobilisation, via an affinity tag, into a spatially defined array on a solid surface. These proteins are expressed in parallel and the cell lysates used to directly capture the tagged proteins onto an affinity capture surface to fabricate a high density protein array on capture member.

Functional assays are performed to assess for example, DNA-binding, protein-binding, small molecule and drug binding and the effects of modifications such as phosphorylation on protein function. The plasmid DNA encoding the positive 'hits' is then sequenced in order to determine the identity of the protein.

**Example 3: Use of differntially expressed protein arrays in identifying proteins which interact with CFTR (Cystic Fibrosis Transmembrane Receptor)**

Intestinal biopsies are obtained from null-CF with the d508 mutant, none-CF and residual-CF (d508 mutant). RNA is then extracted from these libraries and first strand and cDNA libraries are prepared. Subtractive hybrisation is then performed (as already described herein) to generate the following proteome arrays:

1. a subtracted null-CF/none-CF array
2. a subtracted residula-CF/none-CF array
3. a subtracted null-CF/residual-CF array

One or more of these arrays can be probed with one or more peptides derived from CFTR which are known to bind cellular proteins, as a positive control.

**References**

1. Yuan R, Fan S, Achary M, Stewart DM, Goldberg ID, Rosen EM. Cancer Res. (2001) 61(21): 8022-31.

2. Unger MA, Rishi M, Clemmer VB, Hartman JL, Keiper EA, Greshock JD, Chodosh LA, Liebman MN, Weber BL. Breast Cancer Res (2001) 3(5):336-41

3. Alberti I, Barboro P, Barbesino M, Sanna P, Pisciotta L, Parodi S, Nicolo G, Boccardo F, Galli S, Patrone E, Balbi C. J Cell Biochem. (2000) 79(3): 471-85.

4. Fey SJ, Larsen PM Curr Opin Chem Biol (2001) 5(1):26-33

5. Ko L, Cardona GR, Henrion-Caude A, Chin WW. Mol Cell Biol.(2002) 22(1): 357-69

6. Song RX, McPherson RA, Adam L, Bao Y, Shupnik M, Kumar R, Santen RJ. Mol Endocrinol. (2002) 16(1): 116-127.

7. Zhu H, Klemic JF, Chang S, Bertone P, Casamayor A, Klemic KG, Smith D, Gerstein M, Reed MA, Snyder M. Nat Genet (2000) 26(3):283-9

8. Knezevic V, Leethanakul C, Bichsel VE, Worth JM, Prabhu VV, Gutkind JS, Liotta LA, Munson PJ, Petricoin EF 3rd, Krizman DB Proteomics (2001) 1(10):1271-8

9. Sreekumar A, Nyati MK, Varambally S, Barrette TR, Ghosh D, Lawrence TS, Chinnaiyan AM Cancer Res (2001) 61(20):7585-93

10. He, M., Taussig, M.J., Nucleic Acids Research. (2001) 29(15), e73

11. Joos TO, Schrenk M, Hopfl P, Kroger K, Chowdhury U, Stoll D, Schomer D, Durr M, Herick K, Rupp S, Sohn K, Hammerle H Electrophoresis (2000) 21(13):2641-50

12. Zhu, H., Bilgin, M., Bangham, R., Hall, D., Casamayor, A., Bertone, P., Lan, N., Jansen, R., Bidlingmaier, S., Houfek, T., Mitchell, T., Miller, P., Dean, R.A., Gerstein, M., Snyder, M., Science. (2001) 293(5537), 2101-2109

13. PCT patent application no. PCT/GB01/03693. PCT patent application no. PCT/GB01/00395

14. Sambrook, J., Fritsch, E.F., Maniatis, T. CSH Laboratory Press, Cold Spring Harbour, NY 1989

15. Galliciotti G, Schneider H, Wyder L, Vitaliti A, Wittmer M, Ajmo M, Klemenz R. J Membr Biol (2001) 183(3):175-82

16. Valdez BC, Perlaky L, Cai ZJ, Henning D, Busch H. Biotechniques (1998) 24(6): 1032-6

17. BCCP patent application pending publication

18. Gibson L, Holmgreen SP, Huang DC, Bernard O, Copeland NG, Jenkins NA, Sutherland GR, Baker E, Adams JM, Cory S. Oncogene (1996) 13(4): 665-75

19. Nakayama K, Kanzaki A, Hata K, Katabuchi H, Okamura H, Miyazaki K, Fukumoto M, Takebayashi Y. Cancer Lett. (2002) 176(2): 215-23

**Table 1**

Soluble proteins expressed from breast tumour-associated cDNAs after subtractive hybridisation against normal breast tissue cDNAs

| Breast tumour-associated proteins (SwissProt number) | |
|---|---|
| histidine-rich glycoprotein (XM_029591.1) | ATP synthase sununit c isoform 2 (XM_028629) |
| Myoglobin (XM_009949.1) | Actin (BC009978) |
| ribosomal protein S27a (XM_048097.1) | RNA-binding motif, ss interacting protein (NM_002897) |
| alpha gene (AF203815.1) | IMAGE:3510538 (BC008007.1) |
| NADH dehydrogenase subunit 4 (NC_001807.3) | ATP synthase alpha subunit isoform 1 (XM_038872) |
| Crystalline (NM_001885.1) | Proteolipid 68MP homolog (AF054175) |
| NADH dehydrogenase subunit 4L (NC_001807.3) | ATP synthase, H+ transporting (BC011384.1) |
| cytochrome c oxidase subunit I | phosphogluconate dehydrogenase |

(continued)

Soluble proteins expressed from breast tumour-associated cDNAs after subtractive hybridisation against normal breast tissue cDNAs

| Breast tumour-associated proteins (SwissProt number) | |
|---|---|
| (AF346977.1) | (XM_008826) |
| myosin light chain-2 (S69022.1) | hypoxia-inducbile gene 1 (AF145385.1) |
| myosin, light polypeptide, non-sarcomeric (NM_006471.1) | IMAGE:429687 (BC012558) |
| hypothetical protein CDA08 (XM_040811.1) | microsomal glutathione S-transferase 3 (XM_047471.1) |
| sterol carrier protein 2 (XM_001759.4) | myeloid leukaemia factor 1 (XM_017436) |
| ATPase subunit D (ATP6M) (XM_031046) | cathepsin S (XM_041904.1) |
| glyceraldehyde-3-phosphate dehydrogenase (XM_033269) | fatty-acid-Coenzyme A ligase (XM_037131.1) |
| tropomyosin 1 (XM_029494) (BC011359.1) | ferritin, heavy polypeptide 1 |
| ribosomal protein S3A (XM_037459) | small muscle protein, X-linked (XM_010086) |
| KIA0103 gene product (XM_005236) | malate dehydrogenase 1 (BC001484) |
| vitamin A responsive gene JWA (XM_037454) | IMAGE: 3507241 (BC014301) |
| troponin C (XM_037415) | beta-2-microglobulin (AF072097) |
| solute carrier family 25, member 3 (XM_039618) | cytochrome c oxidase subunit Vic (BC000187.1) |
| BCL2-like 2 gene (NM_004050.2) | crystallin, alpha B (NM_001885.1) |
| HSPC055 protein (XM_042290.1) | ribosomal protein L7 (BC014653) |
| ATP synthase coupling factor 6 (XM_009708) | ssDNA-binding protein 1(XM_004810) |
| ribosomal protein L4 (BC014653) | TGF beta inducible nuclear protein (AF077615) |
| Titin (XM_038278) | myomesin (M-protein) 2 (XM_005198.4) |
| calmodulin 2 (XM_031623) | unknown gene (XM005198) |
| lactate dehydrogenase B (BC015122) | proteasome subunit alpha type 2 (NM_002787) |
| TNF alpha-inducible cellular protein (XM_056456) | Similar to unknown gene (LOC91624) |

Further embodiments of the invention are described below.

Embodiment 1. A protein array comprising a surface upon which are deposited at spatially defined locations at least two protein moieties characterised in that said protein moieties represent at least part of a set of proteins which are differentially expressed.

Embodiment 2. A protein array as described in embodiment 1 wherein the set of proteins which are differentially expressed are derived from:

(a) proteins expressed in different cellular compartments, eg the nucleus or the cytoplasm;
(b) proteins that are eventually translocated out of the cell entirely, ie secreted proteins;
(c) proteins whose expression levels alter in response to an external stimulus such as drug treatment; or
(d) proteins whose expression levels alter between healthy and diseased states.

Embodiment 3. A protein array as described in embodiment 2 wherein the set of proteins which are differentially expressed are derived from proteins whose expression levels alter between healthy and diseased states.

Embodiment 4. A protein array as described in embodiment 3 wherein the disease state is cancer, cystic fibrosis, heart disease, neurological disorder, pre or post treatment tissues, kidney disease, multiple sclerosis and degenerative diseases.

Embodiment 5. A method of making a protein array comprising the steps of:

a) providing DNA coding sequences which code for two or more proteins from a set of differentially expressed proteins;
b) expressing said coding sequences to provide individual proteins;
c) purifying said proteins; and
d) depositing said proteins at spatially defined locations on a surface to give an array.

Embodiment 6. A method as described in embodiment 5, wherein steps c) and d) are combined in a single step by the simultaneous purification and isolation of the protein on the array via an incorporated tag.

Embodiment 7. A method as described in embodiment 5 or embodiment 6 wherein step a) includes the use of subtractive hybridisation to identify coding sequences of interest.

Embodiment 8. A method for screening/evaluating the effect of a drug/chemical entity/therapeutic moiety which comprises the step of bringing said drug/chemical entity/therapeutic moiety into contact with an array as defined in any one of embodiments 1 to 4.

Embodiment 9. A method for screening peptides or proteins for the ability to interact selectively with a protein on the array, said method comprising the steps of:

(i) providing an array of differentially expressed proteins,
(ii) contacting the array with compounds or peptides or proteins, and
(iii) identifying the interacting partners.

Embodiment 10. The method of embodiment 8 or embodiment 9, which method comprises the additional step (iv) of quantitating the interaction of the interacting partners.

Embodiment 11. A method for screening compounds or peptides or proteins for the ability to selectively modulate the interaction between a protein and a differentially expressed protein, said method comprising the steps of

(i) providing an array of differentially expressed proteins,
(ii) contacting the array with compounds or peptides or proteins and with other proteins thereof of interest, either simultaneously or in sequence,
(iii) determining whether said interaction is modulated by the presence of said compounds or peptides or proteins.

Embodiment 12. The method of embodiment 11, said method comprising the additional step (iv) of quantitating the degree of modulation of the interaction.

Embodiment 13. The use of an array of differentially expressed proteins as described in any one of embodiments 1 to 4 to capture and measure the simultaneous binding of more than one other protein i.e. protein rafts comprising of two or more proteins.

Embodiment 14. The use of an array of differentially expressed proteins as described in any one of embodiments 1 to 4 as an affinity surface on which to select antibodies from a library of phenotype-genotype-linked antibodies (e. g. phage displayed antibodies).

Embodiment 15. The use of an array of differentially expressed proteins as described in any one of embodiments 1 to 4 for determining the effect of post-translational modifications on the interactions of said proteins with other moieties and/or on the properties of said differentially expressed proteins.

**Claims**

1. A protein array comprising a surface upon which are deposited at spatially defined locations at least two protein moieties **characterised in that** said protein moieties represent at least part of a set of proteins which are differentially expressed.

2. A protein array as claimed in claim 1 wherein the set of proteins which are differentially expressed are derived from:

   (a) proteins expressed in different cellular compartments, eg the nucleus or the cytoplasm;
   (b) proteins that are eventually translocated out of the cell entirely, ie secreted proteins;
   (c) proteins whose expression levels alter in response to an external stimulus such as drug treatment; or
   (d) proteins whose expression levels alter between healthy and diseased states.

3. A protein array as claimed in claim 2 wherein the set of proteins which are differentially expressed are derived from proteins whose expression levels alter between healthy and diseased states.

4. A protein array as claimed in claim 3 wherein the disease state is cancer, cystic fibrosis, heart disease, neurological disorder, pre or post treatment tissues, kidney disease, multiple sclerosis and degenerative diseases.

5. A method of making a protein array comprising the steps of:

   a) providing DNA coding sequences which code for two or more proteins from a set of differentially expressed proteins;
   b) expressing said coding sequences to provide individual proteins;
   c) purifying said proteins; and
   d) depositing said proteins at spatially defined locations on a surface to give an array.

6. A method as claimed in claim 5, wherein steps c) and d) are combined in a single step by the simultaneous purification and isolation of the protein on the array via an incorporated tag.

7. A method as claimed in claim 5 or claim 6 wherein step a) includes the use of subtractive hybridisation to identify coding sequences of interest.

8. A method for screening/evaluating the effect of a drug/chemical entity/therapeutic moiety/peptide/protein/nucleic acid which comprises the step of bringing said drug/chemical entity/therapeutic moiety/peptide/protein/nucleic acid into contact with an array as defined in any one of claims 1 to 4, wherein, optionally, the method comprises:

   i) the use of the array to capture and measure the simultaneous binding of more than one other protein i.e. protein rafts comprising of two or more proteins;
   ii) the use of the array as an affinity surface on which to select antibodies from a library of phenotype-genotype-linked antibodies (e. g. phage displayed antibodies); or
   iii) the use of the array for determining the effect of post-translational modifications on the interactions of said proteins with other moieties and/or on the properties of said differentially expressed proteins.

9. A method according to claim 8 for screening peptides or proteins for the ability to interact selectively with a protein on the array, said method comprising the steps of:

   (i) providing an array of differentially expressed proteins,
   (ii) contacting the array with compounds or peptides or proteins, and
   (iii) identifying the interacting partners.

10. The method of claim 8 or claim 9, which method comprises the additional step (iv) of quantitating the interaction of the interacting partners.

11. The method of claim 10, wherein said quantitating comprises adding the ligand (e.g. DNA, protein, antibody, peptide or small molecule) to replicate arrays at two or more concentrations and determining both the binding affinities and the active concentrations of ligand in the spot.

**12.** A method according to claim 8 for screening compounds or peptides or proteins for the ability to selectively modulate the interaction between a protein and a differentially expressed protein, said method comprising the steps of

(i) providing an array of differentially expressed proteins,
(ii) contacting the array with compounds or peptides or proteins and with other proteins thereof of interest, either simultaneously or in sequence,
(iii) determining whether said interaction is modulated by the presence of said compounds or peptides or proteins.

**13.** The method of claim 12, said method comprising the additional step (iv) of quantitating the degree of modulation of the interaction.

**14.** The use of an array of differentially expressed proteins as claimed in any one of claims 1 to 4 as an affinity surface on which to select antibodies from a library of phenotype-genotype-linked antibodies (e. g. phage displayed antibodies).

**Figure 1.**

Subtracted     Unsubtracted
Cycles:- 18 21 28 33    18 21 28 33

←—G3PDH

**Tumour tissue cDNA**

Subtracted     Unsubtracted
Cycles:- 18 21 28 33    18 21 28 33

**Normal tissue cDNA**

Figure 2.

A                                                    B

Normal breast tissue                  Breast tumour
cDNA probe                            cDNA probe

Europäisches
Patentamt

European
Patent Office

Office européen
des brevets

# EUROPEAN SEARCH REPORT

Application Number

EP 11 15 6690

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 199 43 743 A (JERINI BIO TOOLS GMBH) 15 March 2001 (2001-03-15) * the whole document * | 1-4,8-14 | INV. G01N33/68 |
| Y | US 5 827 658 A (LIANG, B.C.) 27 October 1998 (1998-10-27) * the whole document * | 7 | |
| X | WO 00/07024 A (SMITHKLINE BEECHAM) 10 February 2000 (2000-02-10) | 1-5,8-14 | |
| Y | * the whole document * | 1-14 | |
| X | UTAL A ET AL: "PRELIMINARY EXPRESSION ANALYSIS IN ALZHEIMER'S DISEASE USING SELDI PROTEIN CHIPS", SOCIETY FOR NEUROSCIENCE ABSTRACTS, SOCIETY FOR NEUROSCIENCE, US, vol. 26, no. 1-2, 2000, page 232, XP000989844, ISSN: 0190-5295 * the whole document * | 1-14 | |
| Y | WALTER G ET AL: "PROTEIN ARRAYS FOR GENE EXPRESSION AND MOLECULAR INTERACTION SCREENING", CURRENT OPINION IN MICROBIOLOGY, CURRENT BIOLOGY LTD, GB, vol. 3, no. 3, June 2000 (2000-06), pages 298-302, XP000941229, ISSN: 1369-5274 * the whole document * | 1-14 | TECHNICAL FIELDS SEARCHED (IPC) G01N |
| X | WO 01/57198 A (SENSE PROTEOMIC LIMITED) 9 August 2001 (2001-08-09) * the whole document * | 1-6,8-14 | |

-/--

The present search report has been drawn up for all claims

| Place of search | Date of completion of the search | Examiner |
|---|---|---|
| Munich | 29 April 2011 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 11 15 6690

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHU H ET AL: "Global analysis of protein activities using proteome chips", SCIENCE, AMERICAN ASSOCIATION FOR THE ADVANCEMENT OF SCIENCE,, US, vol. 293, no. 5537, 14 September 2001 (2001-09-14), pages 2101-2105, XP002201608, ISSN: 0036-8075 * the whole document * | 1-14 | |
| X,D | HUI GE: "UPA, a universal protein array system for quantitative detection of protein-protein, protein-DNA, protein-RNA and protein-ligand interactions", NUCLEIC ACIDS RESEARCH, vol. 28, no. 2, 2000, pages 1-7, XP002634647, * the whole document * | 1-14 | |

TECHNICAL FIELDS
SEARCHED      (IPC)

The present search report has been drawn up for all claims

| Place of search | Date of completion of the search | Examiner |
|---|---|---|
| Munich | 29 April 2011 | Moreno de Vega, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...................................................................................

& : member of the same patent family, corresponding
    document

EPO FORM 1503 03.82 (P04C01)

EP 2 339 350 A1

**ANNEX TO THE EUROPEAN SEARCH REPORT
ON EUROPEAN PATENT APPLICATION NO.**

EP 11 15 6690

This annex lists the patent family members relating to the patent documents cited in the above-mentioned European search report.
The members are as contained in the European Patent Office EDP file on
The European Patent Office is in no way liable for these particulars which are merely given for the purpose of information.

29-04-2011

| Patent document cited in search report | | Publication date | Patent family member(s) | | | Publication date |
|---|---|---|---|---|---|---|
| DE 19943743 | A | 15-03-2001 | AU | 7644200 | A | 10-04-2001 |
| | | | CA | 2386607 | A1 | 15-03-2001 |
| | | | CZ | 20020660 | A3 | 16-10-2002 |
| | | | WO | 0118545 | A2 | 15-03-2001 |
| | | | DE | 10082703 | D2 | 14-02-2002 |
| | | | EP | 1212622 | A2 | 12-06-2002 |
| | | | HU | 0203074 | A2 | 28-12-2002 |
| | | | JP | 2003511652 | T | 25-03-2003 |
| | | | PL | 353995 | A1 | 15-12-2003 |
| US 5827658 | A | 27-10-1998 | NONE | | | |
| WO 0007024 | A | 10-02-2000 | AT | 306666 | T | 15-10-2005 |
| | | | DE | 69927691 | D1 | 17-11-2005 |
| | | | DE | 69927691 | T2 | 13-07-2006 |
| | | | EP | 1101114 | A2 | 23-05-2001 |
| | | | ES | 2251210 | T3 | 16-04-2006 |
| WO 0157198 | A | 09-08-2001 | AT | 477489 | T | 15-08-2010 |
| | | | AU | 3037501 | A | 14-08-2001 |
| | | | CA | 2369742 | A1 | 09-08-2001 |
| | | | EP | 1203238 | A2 | 08-05-2002 |
| | | | JP | 2003521922 | T | 22-07-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 0103693 W

- GB 0100395 W

**Non-patent literature cited in the description**

- **YUAN R ; FAN S ; ACHARY M ; STEWART DM ; GOLDBERG ID ; ROSEN EM.** *Cancer Res.,* 2001, vol. 61 (21), 8022-31
- **UNGER MA ; RISHI M ; CLEMMER VB ; HARTMAN JL ; KEIPER EA ; GRESHOCK JD ; CHODOSH LA ; LIEBMAN MN ; WEBER BL.** *Breast Cancer Res,* 2001, vol. 3 (5), 336-41
- **ALBERTI I ; BARBORO P ; BARBESINO M ; SANNA P ; PISCIOTTA L ; PARODI S ; NICOLO G ; BOCCARDO F ; GALLI S ; PATRONE E.** *J Cell Biochem.,* 2000, vol. 79 (3), 471-85
- **FEY SJ ; LARSEN PM.** *Curr Opin Chem Biol,* 2001, vol. 5 (1), 26-33
- **KO L ; CARDONA GR ; HENRION-CAUDE A ; CHIN WW.** *Mol Cell Biol.,* 2002, vol. 22 (1), 357-69
- **SONG RX ; MCPHERSON RA ; ADAM L ; BAO Y ; SHUPNIK M ; KUMAR R ; SANTEN RJ.** *Mol Endocrinol.,* 2002, vol. 16 (1), 116-127
- **ZHU H ; KLEMIC JF ; CHANG S ; BERTONE P ; CASAMAYOR A ; KLEMIC KG ; SMITH D ; GERSTEIN M ; REED MA ; SNYDER M.** *Nat Genet,* 2000, vol. 26 (3), 283-9
- **KNEZEVIC V ; LEETHANAKUL C ; BICHSEL VE ; WORTH JM ; PRABHU VV ; GUTKIND JS ; LIOTTA LA ; MUNSON PJ ; PETRICOIN EF 3RD ; KRIZMAN DB.** *Proteomics,* 2001, vol. 1 (10), 1271-8

- **SREEKUMAR A ; NYATI MK ; VARAMBALLY S ; BARRETTE TR ; GHOSH D ; LAWRENCE TS ; CHINNAIYAN AM.** *Cancer Res,* 2001, vol. 61 (20), 7585-93
- **HE, M. ; TAUSSIG, M.J.** *Nucleic Acids Research,* 2001, vol. 29 (15), 73
- **JOOS TO ; SCHRENK M ; HOPFL P ; KROGER K ; CHOWDHURY U ; STOLL D ; SCHOMER D ; DURR M ; HERICK K ; RUPP S.** *Electrophoresis,* 2000, vol. 21 (13), 2641-50
- **ZHU, H. ; BILGIN, M. ; BANGHAM, R. ; HALL, D. ; CASAMAYOR, A. ; BERTONE, P. ; LAN, N. ; JANSEN, R. ; BIDLINGMAIER, S. ; HOUFEK, T.** *Science,* 2001, vol. 293 (5537), 2101-2109
- **GALLICIOTTI G ; SCHNEIDER H ; WYDER L ; VITALITI A ; WITTMER M ; AJMO M ; KLEMENZ R.** *J Membr Biol,* 2001, vol. 183 (3), 175-82
- **VALDEZ BC ; PERLAKY L ; CAI ZJ ; HENNING D ; BUSCH H.** *Biotechniques,* 1998, vol. 24 (6), 1032-6
- **GIBSON L ; HOLMGREEN SP ; HUANG DC ; BERNARD O ; COPELAND NG ; JENKINS NA ; SUTHERLAND GR ; BAKER E ; ADAMS JM ; CORY S.** *Oncogene,* 1996, vol. 13 (4), 665-75
- **NAKAYAMA K ; KANZAKI A ; HATA K ; KATABUCHI H ; OKAMURA H ; MIYAZAKI K ; FUKUMOTO M ; TAKEBAYASHI Y.** *Cancer Lett.,* 2002, vol. 176 (2), 215-23